# EUROPÄISCHE PATENTSCHRIFT

(11) **EP 2 322 511 B1**
(45) Veröffentlichungstag und Bekanntmachung des Hinweises auf die Patenterteilung: **31.07.2013**
(21) Anmeldenummer: 10192561.8
(22) Anmeldetag: 26.03.2004
(51) Int. Cl.: C07D 235/18

(54) **Verfahren zur Herstellung von Telmisartan**
Process for the production of Telmisartan
Procédé pour la préparation de Telmisartan

(30) Priorität: 31.03.2003 DE 10314702
(43) Veröffentlichungstag der Anmeldung: 18.05.2011
(62) Teilanmeldung aus: 04739071.1
(73) Patentinhaber: Boehringer Ingelheim International GmbH, 55216 Ingelheim am Rhein (DE); Boehringer Ingelheim Pharma GmbH & Co. KG, 55216 Ingelheim am Rhein (DE)
(72) Erfinder: Hauel, Norbert, 88433, Schemmerhofen (DE); Dach, Rolf, 55435, Gau-Algesheim (DE); Heitger, Helmut, 55218, Ingelheim am Rhein (DE); Meyer, Oliver, 55452, Dorsheim (DE)
(74) Vertreter: Simon, Elke Anna Maria

(56) Entgegenhaltungen:
- EP-A- 0 502 314
- MEDERSKI ET AL: "Non-Peptide Angiotensin II Receptor Antagonists: Synthesis and Biological Activity of a Series of Novel 4,5-Dihydro-4-oxo-3H-imidazo [4,5-c]pyridine Derivatives", JOURNAL OF MEDICINAL CHEMISTRY, AMERICAN CHEMICAL SOCIETY. WASHINGTON, US, Bd. 37, Nr. 11, 1994, Seiten 1632-1645, XP002129108, ISSN: 0022-2623
- COTTINEAU B ET AL.: "Synthesis and Hypogylcemic Evaluation of Substituted Pyrazole-4-Carboxylic Acids", BIOORGANIC & MEDICINAL CHEMISTRY LETTERS, Bd. 12, 2002, Seiten 2105-2108, XP002280950,
- BRÖRING, M. ET AL.: "Synthetic aspects of 2,2'-bisdipyrrins", JOURNAL OF PORPHYRINS AND PHTHALOCYANINS, Bd. 5, 2001, Seiten 708-714, XP002628725,

## Beschreibung

### Gebiet der Erfindung

Gegenstand der vorliegenden Erfindung ist ein neues Verfahren zur Herstellung von 4'-[2-n-Propyl-4-methyl-6-(1-methylbenzimidazol-2-yl)benzimidazol-1-ylmethyl]-biphenyl-2-carbonsäure (INN: Telmisartan) Hydrochlorid.

### Hintergrund der Erfindung

Telmisartan ist ein Angiotensin-II-Rezeptor-Antagonist, der zur Behandlung von Bluthochdruck und anderen medizinischen Indikationen geeignet ist, wie in EP-502314 B1 beschrieben. Der Wirkstoff hat folgende Struktur:

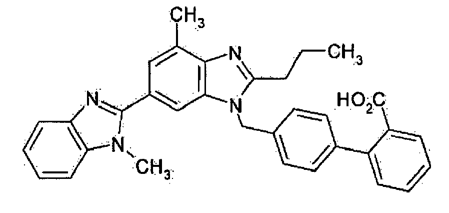

Telmisartan wird im allgemeinen in Form der freien Säure hergestellt und vertrieben. Wie in WO 00/43370 offenbart, liegt kristallines Telmisartan in zwei polymorphen Formen vor, die unterschiedliche Schmelzpunkte haben. Unter Einfluss von Hitze und Feuchtigkeit wandelt sich die niedriger schmelzende polymorphe Form B irreversibel in die höher schmelzende polymorphe Form A um.

Die technische Synthese von Telmisartan erfolgte bisher durch Umsetzung von 2-n-Propyl-4-methyl-6-(1'-methylbenzimidazol-2'-yl)-benzimidazol (I) mit 4'-Brommethyl-biphenyl-2-carbonsäure-tert.butylester (II) und sich anschließender Verseifung nach folgendem Schema 1.

Schema 1:

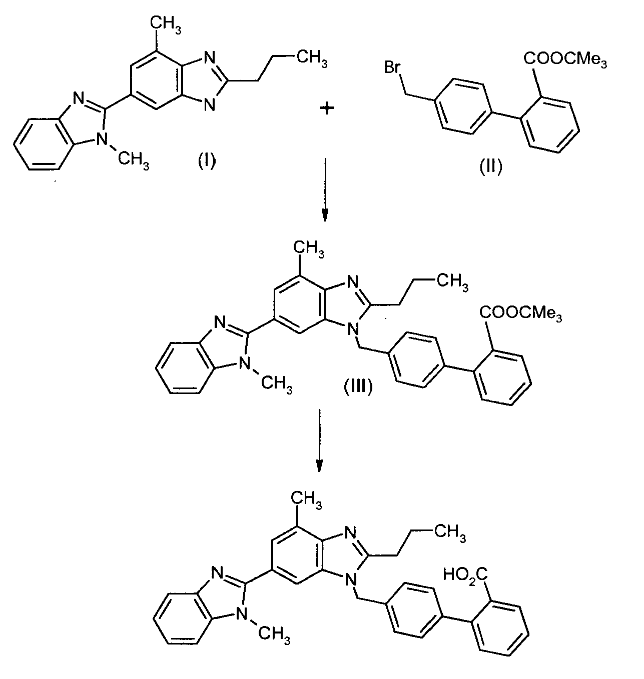

Die Kupplung durch nukleophile Substitution im ersten Reaktionsschritt ist in EP-502314 B1 als Verfahren b) in allgemeiner Weise beschrieben, die Verseifung der tert. Butylestergruppe im Labormaßstab unter Verwendung von Trifluormethylessigsäure wird in der Patentschrift als Beispiel 1 beschrieben. Technisch erfolgt die Verseifung bisher mit konzentrierter wäßriger Bromwasserstoffsäure. Die Übertragung des aus der genannten Patentschrift bekannten Syntheseverfahrens in einen großtechnischen Herstellungsprozess war überraschenderweise nicht problemlos durchführbar. So kann der nach dem bisherigen Verfahren hergestellte Wirkstoff in hinreichender Qualität erst nach Durchlauf mehrerer Prozeßstufen (das Rohprodukt weist erst nach zweimaligem Umkristallisieren die erforderliche Reinheit auf) erhalten werden, wobei beim Isolieren der Substanz sehr lange Zentrifugier- und Trocknungszeiten nötig sind. Das gemäß Schema 1 großtechnisch synthetisierte Telmisartan fällt nach Aufarbeitung in Form eines Produktes an, welches zur abschließenden Reinigung einem zweiten Kristallisationsschritt unterworfen werden muß. Bei besagtem, zwingend erforderlichen Kristallisationsschritt führte die Morphologie des auskristallisierenden Endprodukts zu unvorhergesehenen Schwierigkeiten.

Das in Form langer Nadeln als Feststoff ausfallende Produkt kann nur schwer filtriert, gewaschen und isoliert werden, zeichnet sich ferner aufgrund des Einschlusses von Lösungsmittel durch eine sehr lange Trocknungszeit aus und bildet beim Trocknungsprozess große, sehr harte Brocken. Eine Zerkleinerung dieser Brocken führt zu einem trockenen Pulver welches eine hohe Tendenz zur elektrostatischen Aufladung zeigt und praktisch nicht rieselfähig ist.

Obige, nachteilige Eigenschaften eines Produkts erweisen sich bei der großtechnischen Herstellung einer Verbindung stets als äußerst hinderlich, da sie deren reproduzierbare Bereitstellung in größerer Menge und hoher Reinheit nur unter großen Schwierigkeiten oder zusätzlichem, hohem technischen Aufwand zulassen.

Mederski et al (J Med Chem 37: 1632-1645, 1994) und Bröring et al. (Journal of Prophyrins and Phthalocyanins 5: 708-714, 2001) erwähnen u.a. eine basische Hydrolyse von substituiertem 2-Cyanobiphenyl-Verbindungen zu entsprechenden Säuren.

Es ist Aufgabe der vorliegenden Erfindung, ein alternatives Verfahren zur Herstellung von Telmisartan bereitzustellen, welches großtechnisch anwendbar ist und eine einfache Aufarbeitung, Reinigung und Isolierung von Telmisartan ohne die vorstehend genannten Nachteile erlaubt.

### Kurze Zusammenfassung der Erfindung

Überraschenderweise wurde gefunden, daß aus verfahrenstechnischer Sicht die Umsetzung von 2-n-Propyl-4-methyl-6-(1'-methylbenzimidazol-2'-yl)-benzimidazol 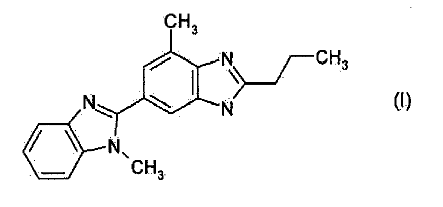 mit einer Verbindung der allgemeinen Formel 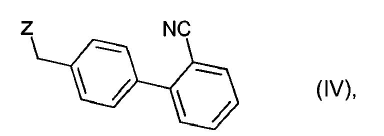 in der Z eine Austrittsgruppe wie ein Halogenatom, beispielsweise ein Chlor-, Brom-oder lodatom, oder eine substituierte Sulfonyloxygruppe, beispielsweise eine Methansulfonyloxy-, Phenylsulfonyloxy- oder p-Toluolsulfonyloxygruppe, darstellt, wobei die Verbindung 2-Cyano-4'-[2"-n-propyl-4"-methyl-6"-(1"'-methylbenzimidazol-2"'-yl)benzimidazol-1 "-ylmethyl]biphenyl 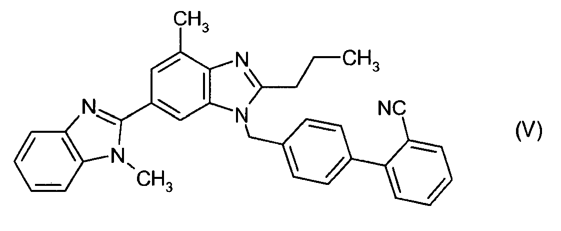 erhalten wird, die gewünschtenfalls einer Aufarbeitung unterzogen wird (Stufe (a)), und sich anschließender Hydrolyse der Nitril- zur Säurefunktion (Stufe (b)) sowie gewünschtenfalls Überführung der Verbindung (V) während der Aufarbeitung in das Hydrochlorid erhebliche Vorteile gegenüber der in Schema 1 dargestellten Synthese aufweist, insbesondere die für die großtechnische Herstellung nach dem konventionellen Verfahren vorstehend erwähnten Nachteile nicht aufweist.

### Detaillierte Beschreibung der Erfindung

### Stufe (a):

Die Umsetzung der Verbindung (I) mit einer Verbindung der allgemeinen Formel (IV), in der Z vorzugsweise ein Halogenatom, insbesondere das Bromatom darstellt, wird zweckmäßigerweise in einem Lösungsmittel oder Lösungsmittelgemisch wie Methylenchlorid, Diethylether, Tetrahydrofuran, Dioxan, Dimethylsulfoxid, Dimethylformamid, Dimethylacetamid, Dimethylformamid/tert.-Butanol, Dimethylacetamid/tert.-Butanol, Toluol und Benzol gegebenenfalls in Gegenwart eines säurebindenden Mittels wie Natriumcarbonat, Kaliumcarbonat, Natriumhydroxid, Kaliumhydroxid, Natriummethylat, Kaliummethylat, Kalium-tert.-pentylat, Kalium-tert.butylat, Kalium-n-butylat, Natriumhydrid, Triethylamin oder Pyridin, wobei die beiden letzteren auch als Lösungsmittel verwendet werden können, beispielsweise bei einer Temperatur zwischen 0 und 100°C durchgeführt. Die Base wird, sofern es sich um einen Feststoff handelt, vorzugsweise in pulverisierter Form eingesetzt.

Vorzugsweise wird die Umsetzung der Verbindung (I) mit einer Verbindung der allgemeinen Formel (IV) in einem Lösungsmittel oder Lösungsmittelgemisch ausgewählt aus Dimethylsulfoxid, Dimethylformamid, Dimethylacetamid, Dimethylformamid/tert.-Butanol und Dimethylacetamid/tert.-Butanol in Gegenwart von Natriumhydroxid, Kaliumhydroxid oder Kalium-tert.butylat bei einer Temperatur zwischen 0 und 30°C durchgeführt.

Besonders bevorzugt wird die Umsetzung der Verbindung (I) mit einer Verbindung der allgemeinen Formel (IV) in Dimethylacetamid oder Dimethylacetamid/tert.-Butanol in Gegenwart von Kaliumhydroxid bei einer Temperatur zwischen 0 und 20°C durchgeführt.

Aufarbeitung:
Nach Beendigung der Umsetzung wird das Lösungsmittel entfernt, beispielsweise im Wasserstrahlvakuum abdestilliert, der Rückstand mit einem Lösungsmittel behandelt, in dem das Nitril (V) nur begrenzt bzw. in der Wärme mäßig löslich ist, beispielsweise mit einem Alkohol wie Methanol, Ethanol, n-Propanol, i-Propanol, n-Butanol oder i-Butanol, mit einem aromatischen Kohlenwasserstoff wie Benzol oder Toluol, mit einem etherischen Lösungsmittel wie Diethylether, Tetrahydrofuran, Dioxan oder tert. Butylmethylether, wobei die etherischen Lösungsmittel und insbesondere tert. Butylmethylether bevorzugt sind, oder auch mit Wasser, die gegebenenfalls nach Abkühlen auf 10 bis 20°C ausfallenden Kristalle abgesaugt und zunächst mit dem verwendeten Lösungsmittel und anschließend mit Wasser nachgewaschen. Erforderlichenfalls wird das Produkt bei erhöhter Temperatur, beispielsweise bei 50-100°C, im Vakuumtrockenschrank getrocknet. Das Nitril (V) wird in der Regel in ausgezeichneten Ausbeuten zwischen 80 und 90 % der Theorie und einer exzellenten Qualität (Reinheit nach HPLC > 99,5%) erhalten.

### Stufe (b):

Die sich anschließende Hydrolyse der Nitrilfunktion in eine Carboxygruppe erfolgt zweckmäßigerweise in Wasser, in einem organischen Lösungsmittel oder in einer Mischung aus einem organischen Lösungsmittel mit Wasser, wobei das organische Lösungsmittel beispielsweise Methanol, Ethanol, n-Propanol, Isopropanol, Tetrahydrofuran, Dioxan, Ethylenglykol, Propylenglycol, Diglyme, Dimethylsulfoxid oder Diethylenglycolmonomethylether sein kann, in Gegenwart einer Base wie Lithiumhydroxid, Natriumhydroxid, Kaliumhydroxid, Cäsiumhydroxid oder Calciumhydroxid oder deren Anhydride bei Temperaturen zwischen 80 und 200°C, wobei das für die Reaktion benötigte Wasser auch Bestandteil eines der verwendeten Reagenzien, sein kann oder unter den Reaktionsbedingungen aus den Reagenzien generiert werden kann, etwa aus einem der vorstehend genannten Alkalihydroxide.

Vorzugsweise erfolgt die Hydrolyse der Nitrilfunktion in einem hochsiedenden Lösungsmittelsystem ausgewählt aus Ethylenglykol/Wasser und Propylenglycol/Wasser, in Gegenwart einer Base, wobei Kaliumhydroxid besonders geeignet ist, bei Temperaturen zwischen 140 und 200°C, insbesondere bei einer Temperatur zwischen 155 und 185°C.

Aufarbeitung:
Nach Beendigung der Umsetzung wird das Lösungsmittel entfernt, beispielsweise im Wasserstrahlvakuum abdestilliert, der Rückstand mit Wasser verdünnt und mit Salzsäure aufgenommen wird, beispielsweise mit 5 bis ca. 32%iger (konz.) Salzsäure, vorzugsweise mit 5 bis 20%iger Salzsäure, wobei Telmisartan Hydrochlorid auskristallisiert. Die Kristallsuspension wird erforderlichenfalls auf 10 bis 25°C abgekühlt und kann für gewisse Zeit, beispielsweise bis zu 3 Stunden, bei dieser Temperatur gerührt werden. Nach Absaugen der Kristalle wird mit Wasser gewaschen und erforderlichenfalls im Vakuumtrockenschrank bei erhöhter Temperatur, beispielsweise bei 50 bis 120°C, getrocknet.

Aus dem Telmisartan Hydrochlorid kann auf übliche Weise Telmisartan in der Säure-Form freigesetzt werden, etwa durch Titration mit wässriger Alkalihydroxidlösung. Beispielsweise erfolgt die Freisetzung der Säureform in Analogie zu dem in der WO 0043370 (Seite 3, Zeile 6 bis S. 4, Zeile 38, sowie die Beispiele 1 bis 3) beschriebenen Verfahren.

Das erfindungsgemäße Verfahren weist für die großtechnische Herstellung unter anderem folgende besonders zu erwähnende Vorteile auf:
- Verbindungen der Formel (IV), insbesondere das 4'-Brommethyl-2-cyanobiphenyl, werden großtechnisch hergestellt und sind kostengünstig kommerziell erhältlich;
- die Kupplung der Komponenten (I) und (IV) gemäß Stufe (a) lässt sich in hoher Konzentration und entsprechend hohem Durchsatz durchführen, wobei durch Aufarbeitung insbesondere unter Verwendung von tert. Butylmethylether das Nitril (V) als leicht filtrier- und waschbarer Niederschlag erhalten wird, wodurch sich weitere aufwändige Aufarbeitungsschritte erübrigen;
- das Nitril (V) wird in ausgezeichneten Ausbeuten zwischen 80 und 90 % der Theorie und einer exzellenten Qualität (Reinheit nach HPLC > 99,5%) erhalten;
- die Verseifung des Nitrils (V) in Stufe (b) erfolgt ebenfalls in ausgezeichneten Ausbeuten > 95% der Theorie;
- das Endprodukt Telmisartan kann entweder als Zwitterion oder, bevorzugt, durch Fällung mit Salzsäure als leicht filtrierbares und damit einfach zu reinigendes Hydrochlorid isoliert werden.

In erfindungsgemäß besonders bevorzugter Art und Weise wird wie folgt vorgegangen:
**Stufe (a):** Alle Mengenangaben beziehen sich auf eine Ansatzgröße von 0,1 Mol der Verbindung (I) und sind bei Änderung der Ansatzgröße mit einem entsprechenden Faktor zu multiplizieren.

In einem geeignet dimensionierten Reaktionsgefäß werden pro 0,1 Mol der Verbindung (I) 50 bis 200 ml Lösungsmittel, vorzugsweise 80 bis 120 ml, vorgelegt, die Verbindung (I) in dem Lösungsmittel suspendiert und unter Rühren 0,1 bis 0,2 Mol, Base, vorzugsweise 0,102 bis 0,12 Mol, portionsweise unter Rühren hinzugefügt, wobei die Temperatur zwischen 10 und 50°C, vorzugsweise 15 bis 30°C, gehalten und nach Beendigung der exothermen Reaktion weitere bis zu 3 Stunden bei dieser Temperatur gerührt werden kann. Der Ansatz wird auf ca. 0 - 10°C, beispielsweise ca. 5°C, abgekühlt und dann eine Mischung aus 0,1 bis 0,2 Mol einer Verbindung der allgemeinen Formel (IV), vorzugsweise 0,100 bis 0,12 Mol, mit 50 bis 200 ml Lösungsmittel (pro 0,1 Mol der Verbindung (IV)) bei 10 bis 30°C, vorzugsweise bei ca. 20°C zugetropft. Die Reaktionsmischung wird gegebenenfalls durch Eisbadkühlung bei ca. 0 - 20°C, vorzugsweise 5 bis 10°C, gehalten. Danach kann mit einigen ml Lösungsmittel nachgespült und anschließend noch bis zu 3 Stunden bei 0 - 20°C gerührt werden.

In einer weiteren Ausführungsform wird die Base in 30 bis 100 ml Lösungsmittel, vorzugsweise Dimethylformamid, Dimethylacetamid, Dimethylformamid/tert.-Butanol oder Dimethylacetamid/tert.-Butanol, vorgelegt, bei 10 bis 30°C, beispielsweise etwa 20°C bis zu einer Stunde gerührt und anschließend eine Suspension der Verbindung (I) in 30 bis 100 ml Lösungsmittel bei dieser Temperatur langsam zudosiert. Alle weiteren Schritte entsprechen denjenigen der vorstehenden Ausführungsform. Die angegebenen Lösungsmittelgemische werden in einem Volumenverhältnis von Amid : tert.-Butanol = 10 : 1 bis 2,5 : 1, beispielsweise 5 : 1, eingesetzt.

Aufarbeitung:
Das Lösungsmittel wird zweckmäßigerweise unter erniedrigtem Druck, beispielsweise im Wasserstrahlvakuum, weitgehend abdestilliert, wobei das Produkt auskristallisiert. Nach Abkühlen des Rückstands auf ca. 40 bis 80°C, vorzugsweise etwa 60°C, wird mit 100 bis 300 ml Lösungsmittel (pro 0,1 Mol Ansatzgröße, bezogen auf Verbindung (I)), vorzugsweise tert. Butylmethylether, verdünnt und bis zu 5 Stunden ohne Energiezufuhr gerührt. Der Ansatz wird auf 0 bis 30°C, vorzugsweise 15 - 20°C, abgekühlt und nochmals bis zu 5 Stunden bei dieser Temperatur gerührt. Die Kristalle werden abgesaugt, mit 50 bis 150 ml des Lösungsmittels und anschließend mit 200 bis 300 ml Wasser portionsweise gewaschen. Das Produkt wird im Vakuumtrockenschrank bei 50 bis 100°C, vorzugsweise etwa 60°C getrocknet.

**Stufe (b):** Alle Mengenangaben beziehen sich, soweit nicht anders angegeben, auf eine Ansatzgröße von 0,05 Mol der Verbindung (V) und sind bei Änderung der Ansatzgröße mit einem entsprechenden Faktor zu multiplizieren.

0,05 Mol der Verbindung (V), 200 bis 300 ml des organischen Lösungsmittels, 0,5 bis 5 ml Wasser und 0,3 bis 0,5 Mol der Base werden zusammengegeben und bis zur Siedetemperatur des verwendeten Lösungsmittelsystems erhitzt, im Fall der bevorzugten Ethylenglykol/Wasser-Mischung auf 140 bis 200 °C, vorzugsweise auf 155 bis 185°C. Der Ansatz wird bis zu 24 Stunden bei dieser Temperatur gerührt.

In einer weiteren Ausführungsform wird 0,361 Mol des Nitrils (V) in 1,5 bis 2 L des organischen Lösungsmittels, vorzugsweise Ethylenglykol, vorgelegt, 25 bis 50 ml Wasser und 2,5 bis 3 Mol der Base zugegeben und unter Rühren für bis zu 24 Stunden auf 140 bis 200 °C, vorzugsweise auf 155 bis 185°C, erhitzt. Alle weiteren Schritte entsprechen denjenigen der vorstehenden Ausführungsform.

Aufarbeitung:
Die Mengenangaben beziehen sich auf eine Ansatzgröße von 0,05 Mol der Verbindung (V).
Das Lösungsmittel wird zweckmäßigerweise unter vermindertem Druck entfernt, beispielsweise im Wasserstrahlvakuum abdestilliert, der Rückstand mit 30 bis 100 ml Wasser, vorzugsweise etwa 5 ml, verdünnt und in eine Mischung aus 100 bis 150 ml Wasser (vorzugsweise etwa 125 ml) und 40 bis 60 ml (vorzugsweise etwa 50 ml) konz. Salzsäure (ca. 32%ig) eingerührt, wobei mit etwas Wasser nachgespült werden kann. Das auskristallisierende Telmisartan Hydrochlorid wird auf 10 bis 25°C abgekühlt und wird bis zu 3 Stunden bei dieser Temperatur gerührt. Nach Absaugen der Kristalle wird mit 50 bis 200 ml Wasser gewaschen und im Vakuumtrockenschrank bei 50 bis 120°C getrocknet.

Die nachfolgenden Beispiele dienen der Illustration der Erfindung und beziehen sich auf exemplarische Ausführungsformen der erfindungsgemäßen Syntheseverfahren zur Herstellung von Telmisartan, ohne jedoch die Erfindung auf deren Inhalt zu beschränken.

### Beispiel 1

### 2-Cyano-4'-[2"-n-propyl-4"-methyl-6"-(1"'-methylbenzimidazol-2"'-yl)benzimidazol-1"-ylmethyl]biphenyl

32,24 g 2-n-Propyl-4-methyl-6-(1'-methylbenzimidazol-2'-yl)-benzimidazol x H₂O werden in 100 ml Dimethylacetamid (DMA) vorgelegt, unter Rühren 11,8 g Kalium-tert.butylat bei ca. 20°C portionsweise eingetragen und anschließend eine Stunde bei etwa 20°C gerührt. Der Ansatz wird auf 5°C abgekühlt und dann eine Mischung aus 28,6 g 4-Brommethyl-2'-cyano-biphenyl und 95 ml DMA (gelöst bei ca. 20°C) während ca. 30 Minuten zugetropft. Die Temperatur der Reaktionsmischung wird durch Eisbadkühlung bei ca. 5 ―10°C gehalten. Danach wird mit 5 ml DMA nachgespült und es wird weitere 1,5 Stunden bei 5 ―10°C gerührt.

Das Lösungsmittel wird unter Wasserstrahlvakuum weitgehend abdestilliert, wobei das Produkt auskristallisiert. Der Rückstand wird auf 60°C abgekühlt, mit 230 ml tert. Butylmethylether verdünnt und 1 Stunde ohne Energiezufuhr gerührt, dann auf 15 ― 20°C abgekühlt und noch 1 Stunde bei dieser Temp. gerührt. Die Kristalle werden abgesaugt, mit 100 ml tert. Butylmethylether, dann mit 250 ml Wasser portionsweise gewaschen und danach im Vakuumtrockenschrank bei 80°C getrocknet.
Ausbeute: 43,3 g (87,5% d. Th.).
Fp.:196 ― 197°C
HPLC: > 99,9 %

### Beispiel 2

### 2-Cyano-4'-[2"-n-propyl-4"-methyl-6"-(1"'-methylbenzimidazol-2"'-yl)benzimidazol-1"-ylmethyl]biphenyl

32,24 g 2-n-Propyl-4-methyl-6-(1'-methylbenzimidazol-2'-yl)-benzimidazol x H₂O werden in 100 ml DMA vorgelegt, unter Rühren 6,9 g Kaliumhydroxid (Pulver) bei ca. 20°C portionsweise eingetragen und anschließend eine Stunde bei etwa 20 bis 25°C gerührt. Der Ansatz wird auf 5°C abgekühlt und dann 28,6 g 4-Brommethyl-2'-cyano-biphenyl in 95 ml DMA (gelöst bei ca. 20°C) während ca. 30 Minuten zugetropft. Die Temperatur der Reaktionsmischung wird durch Eisbadkühlung bei ca. 5 ―10°C gehalten. Danach wird mit 5 ml DMA nachgespült und es wird weitere 1,5 Stunden bei 5 ―10°C gerührt.

Das Lösungsmittel wird unter Wasserstrahlvakuum weitgehend abdestilliert, wobei das Produkt auskristallisiert. Der Rückstand wird auf 60°C abgekühlt, mit 225 ml tert. Butylmethylether verdünnt und 1 Stunde ohne Energiezufuhr gerührt, dann auf 15 ― 20°C abgekühlt und noch 1 Stunde bei dieser Temp. gerührt. Die Kristalle werden abgesaugt, mit 100 ml tert. Butylmethylether, dann mit 250 ml Wasser portionsweise gewaschen und danach im Vakuumtrockenschrank bei 80°C getrocknet.
Ausbeute: 40,45 g (81,7% d. Th.).
Fp.:196 ― 197°C
HPLC: > 99,9 %

### Beispiel 3

### 2-Cyano-4'-[2"-n-propyl-4"-methyl-6"-(1"'-methylbenzimidazol-2"'-yl)benzimidazol-1"-ylmethyl]biphenyl

6,9 g Kaliumhydroxid (Pulver) werden in 50 ml DMA vorgelegt, 15 Minuten bei 20 bis 25°C gerührt und dann eine Suspension von 32,24 2-n-Propyl-4-methyl-6-(1'-methylbenzimidazol-2'-yl)-benzimidazol x H₂O in 50 ml DMA bei 20 bis 25°C zudosiert. Nach Ende der Zudosierung werden die Gefäße mit 10 ml DMA nachgespült und anschließend noch eine Stunde bei 20 bis 25°C gerührt. Der Ansatz wird auf 5°C abgekühlt und dann 28,6 g 4-Brommethyl-2'-cyano-biphenyl in 95 ml DMA (gelöst bei ca. 20°C) zudosiert. Die Temperatur der Reaktionsmischung wird durch Eisbadkühlung bei ca. 5 ―10°C gehalten. Danach wird mit 5 ml DMA nachgespült und es wird eine weitere Stunde bei 5 ―10°C gerührt.

Das Lösungsmittel wird unter Wasserstrahlvakuum weitgehend abdestilliert, wobei das Produkt auskristallisiert. Der Rückstand wird auf 60°C abgekühlt, mit 250 ml tert. Butylmethylether verdünnt und 2 Stunden ohne Energiezufuhr gerührt. Die Kristalle werden abgesaugt, mit 100 ml tert. Butylmethylether, dann mit 250 ml Wasser portionsweise gewaschen und danach im Vakuumtrockenschrank bei 80°C getrocknet.
Ausbeute: 43,37 g (87,5% d. Th.).
Fp.:196 ― 198°C
HPLC: 99,1 %

### Beispiel 4

### 2-Cyano-4'-[2"-n-propyl-4"-methyl-6"-(1"'-methylbenzimidazol-2"'-yl)benzimidazol-1"-ylmethyl]biphenyl

53,4 g Kaliumhydroxid (Pulver) werden in 385 ml DMA vorgelegt und dann eine Suspension von 248,25 g 2-n-Propyl-4-methyl-6-(1'-methylbenzimidazol-2'-yl)-benzimidazol x H₂O in 385 ml DMA bei 20 bis 25°C zudosiert. Nach Ende der Zudosierung werden die Gefäße mit 77 ml DMA nachgespült und anschließend noch eine Stunde bei 20 bis 25°C gerührt. Der Ansatz wird auf 5°C abgekühlt und dann 209,5 g 4-Brommethyl-2'-cyano-biphenyl in 731,5 ml DMA (gelöst bei ca. 20°C) zudosiert. Die Temperatur der Reaktionsmischung wird durch Eisbadkühlung bei ca. 5 ―10°C gehalten. Danach wird mit 38,5 ml DMA nachgespült und es wird eine weitere Stunde bei 5 ―10°C gerührt.

Das Lösungsmittel wird unter Wasserstrahlvakuum weitgehend abdestilliert, wobei das Produkt auskristallisiert. Der Rückstand wird auf 60°C abgekühlt, mit 1925 ml tert. Butylmethylether verdünnt und 2 Stunden ohne Energiezufuhr gerührt. Die Kristalle werden abgesaugt, mit 770 ml tert. Butylmethylether/DMA = 9:1, dann mit 1925 ml Wasser und zweimal mit je 250 ml tert. Butylmethylether/portionsweise gewaschen und danach im Vakuumtrockenschrank bei 80°C getrocknet.
Ausbeute: 322,15 g (84,4% d. Th.).
Fp.:197 ― 198,5°C
HPLC: 99,6%

### Beispiel 5: Telmisartan x HCI

25 g 2-Cyano-4'-[2"-n-propyl-4"-methyl-6"-(1 "'-methylbenzimidazol-2"'-yl)benzimidazol-1"-ylmethyl]biphenyl, 250 ml Ethylenglykol, 0,9 ml Wasser und 24,75 g Ätzkali (>85%ig) werden zusammen gegeben und unter Rühren auf 160°C erhitzt. Der Ansatz wird 13,5 Stunden bei dieser Temperatur gerührt.

Das Lösungsmittel im Wasserstrahlvakuum weitgehend abdestilliert, der Rückstand auf 100°C abgekühlt, mit 50 ml Wasser verdünnt und in eine Mischung aus 125 ml Wasser und 50 ml konz. Salzsäure (ca. 32%ig) eingerührt, wobei mit 50 ml Wasser nachgespült wird. Das auskristallisierende Telmisartan Hydrochlorid wird auf 15 bis 20°C abgekühlt und ca. 1 Stunde bei dieser Temperatur gerührt. Nach Absaugen der Kristalle wird mit 100 ml Wasser gewaschen und im Vakuumtrockenschrank bei 100°C getrocknet.
Ausbeute: 27,3 g (98,2% d. Th.).
HPLC: 99,9%.

### Beispiel 6: Telmisartan x HCI

179 g 2-Cyano-4'-[2"-n-propyl-4"-methyl-6"-(1"'-methylbenzimidazol-2"'-yl)benzimidazol-1"-ylmethyl]biphenyl werden in 1611 ml Ethylenglykol vorgelegt, 32,5 ml Wasser und 178,7 g Kaliumhydroxid (Pulver) zugegeben und unter Rühren auf 150 bis 160°C erhitzt. Der Ansatz wird ca. 15 Stunden bei dieser Temperatur gerührt und dann auf 100°C abgekühlt.

Das Lösungsmittel im Wasserstrahlvakuum weitgehend abdestilliert, der Rückstand auf 100°C abgekühlt, mit 358 ml Wasser verdünnt und in eine Mischung aus 716 ml Wasser und 358 ml konz. Salzsäure (ca. 32%ig) eingerührt, wobei mit 179 ml Wasser nachgespült wird. Das auskristallisierende Telmisartan Hydrochlorid wird eine Stunde bei 60°C gerührt, auf 15 bis 20°C abgekühlt und ca. eine weiter Stunde bei dieser Temperatur gerührt. Nach Absaugen der Kristalle wird mit 716 ml Wasser gewaschen und im Vakuumtrockenschrank bei 100°C getrocknet.
Ausbeute: 192,1 g (96,5% d. Th.).
HPLC: 99,9%

### Beispiel 7: Telmisartan

5,51 g Telmisartan x HCl werden in 50 ml 40%iger Essigsäure unter Rückflusskochen gelöst. Anschließend wird die braune Lösung heiß über 1,1 g Kohle filtriert, mit 2,5 ml 40%iger Essigsäure gewaschen und das hellbraune Filtrat unter Rühren bei 80 ―90°C tropfenweise mit 2,5 ml 4N NaOH versetzt. Das Telmisartan kristallisiert aus, die Suspension wird mit 30 ml Wasser verdünnt und langsam auf Raumtemperatur abgekühlt. Das Telmisartan wird abgesaugt und mit 50 ml Wasser gewaschen. Das Telmisartan wird im Vakuumtrockenschrank bei 80°C getrocknet.
Ausbeute: 4,80 g (93,3% d. Th.)

## Patentansprüche

1. Verfahren zur Herstellung von Telmisartan Hydrochlocid, in dem
(a) 2-n-Propyl-4-methyl-6-(1'-methylbenzimidazol-2'-yl)-benzimidazol 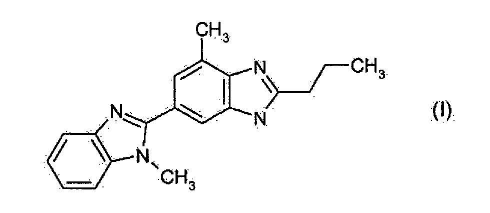 mit einer Verbindung der allgemeinen Formel 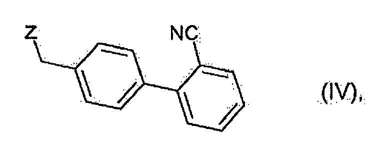 in der Z eine Austrittsgruppe darstellt, umgesetzt wird und die so erhaltene Verbindung gewünschtenfalls einer Aufarbeitung unterzogen wird,
(b) die Cyanogruppe der so erhaltenen Verbindung 2-Cyano-4'-[2"-n-propyl-4"-methyl-6"-(1"-methylbenzimidazol-2"'-yl)benzimidazol-1"-ylmethyl]biphenyl 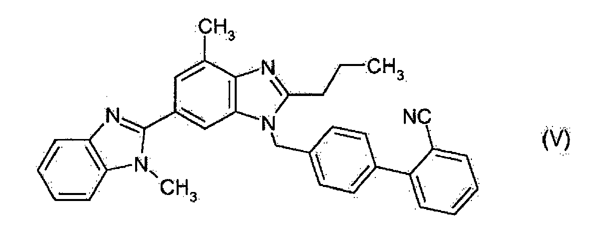 anschließend durch Hydrolyse in Wasser, in einem organischen Lösungsmittel oder in einer Mischung aus einem orgänischen Lösungsmittel mit Wasser in Gegenwart einer Base bei Temperaturen zwischen 80 und 200°C in die Saurefunktion übergeführt wird
und das so erhaltene Telmisartan in das Hydrochlorid übergeführt wird.

2. Verfahren gemäß Anspruch 1, **dadurch gekennzeichnet, dass** die Hydrolyse in einem hochsiedenden Lösungsmiftelsystem ausgewählt aus Ethylenglykol/Wasser und Propylenglycol/Wasser bei Temperaturen zwischen 140 und 200 °C durchgeführt wird.

3. Verfahren gemäß Anspruch 1, **dadurch gekennzeichnet, dass** die Hydrolyse in Gegenwart einer Base ausgewählt aus Lithiumhydroxid, Natriumhydroxid, Kaliumhydroxid, Cäsiumhydroxid und Calciumhydroxid oder deren Anhydride durchgeführt wird.

4. Verfahren gemäß Anspruch 1, **dadurch gekennzeichnet dass** Z ein Halogenatom, vorzugsweise das Bromatom, oder eine substituierte Sulfonyfoxygruppe darstellt.

5. Verfahren gemäß Anspruch 1, **dadurch gekennzeichnet, dass** Schritt (a) in einem Lösungsmittel oder Lösungsmittelgemisch ausgewählt aus Methylenchlorid, Diethylether, Tetrahydrofuran, Dioxan, Dimethylsulfoxid, Dimethylformamid, Dimethylacetamid, Dimethylformamid/tert. -Butanol, Dimethylacetamid/tert.-Butanol, Toluol und Benzol gegebenenfalls in Gegenwart eines säurebindenden Mittels durchgeführt wird.

6. Verfahren gemäß Anspruch 5, **dadurch gekennzeichnet, dass** das säurebindende Mittel ausgewählt ist aus Natriumcarbonat, Kaliumcarbonat, Natriumhydroxid, Kaliumhydroxid, Natriummethylat, Kaliummethylat, Kalium-tert.-pentylat, Kaliumtert. butylaf, Kalium-n-butylat, Natriumhydrid, Trimethylamin und Pyridin.

7. Verfahren gemäß Anspruch 5, **dadurch gekennzeichnet, dass** Schritt (a) bei einer Temperatur zwischen 0 und 140°C durchgeführt wird.

8. Verfahren gemäß einem der Ansprüche 1 bis 7, **dadurch gekennzeichnet, dass** in Schritt (a) die Umsetzung der Verbindung (I) mit einer Verbindung der allgemeinen Formel (IV) in einem Lösungsmittel oder Lösungsmittelgemisch ausgewählt aus Dimethylsulfoxid, Dimethylformamid, Dimethylacetamid, Dimethylformamid/tert.-Butanol und Dimethylacetamid/tert.-Butanol in Gegenwart von Natriumhydroxid, Kaliumhydroxid oder Kalium-tert.butylat bei einer Temperatur zwischen 0 und 30°C durchgeführt wird.

9. Verfahren gemäß einem der Ansprüche 1 bis 8, **dadurch gekennzeichnet, dass** nach Durchführung von Schritt (a) das Lösungsmittel entfernt wird, der Rückstand mit einem Lösungsmittel, in dem das Nitril (V) nur begrenzt bzw. in der Wärme mäßig löslich ist, behandelt wird, die gegebenenfalls nach Abkühlen ausfallenden Kristalle abgesaugt und gegebenenfalls gewaschen werden und das Produkt gewünschtanfälls bei erhöhter Temperatur getrocknet wird.

10. Verfahren gemäß Anspruch 9', **dadurch gekennzeichnet, dass** das Lösungsmittel zur Behandlung des Rückstands ein Alkohol, ein aromatischer Kohlenwasserstoff; ein Ether oder Wasser ist.

11. Verfahren zur Hydrolyse der Verbindung 2-Cyano-4'-[2"-n-propyl-4"-methyl-6"-(1"'-methylbenzimidazol-2'"-yl)benzimidazol-1"-ylmethyl]biphehyl der Formel (V) gemäß Anspruch 1 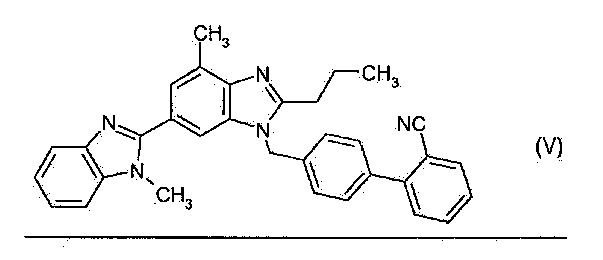 **dadurch gekennzeichnet, dass** die Cyanogruppe der Verbindung (V) in Wasser, in einem organischen Lösungsmittel oder in einer Mischung aus einem organischen Lösungsmittel mit Wasser in Gegenwart einer Base bei Temperaturen zwischen 80 und 200°C in die Säurefunktion überfährt wird.

## Claims

1. Process for preparing telmisartan hydrochloride, wherein
(a) 2-n-propyl-4-methyl-6-(1'-methylbenzimidazol-2'-yl)-benzimidazole 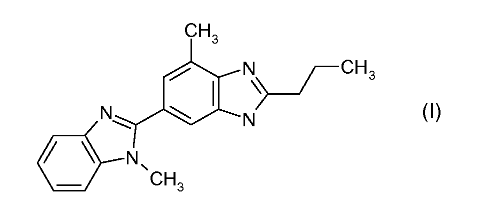 is reacted with a compound of general formula 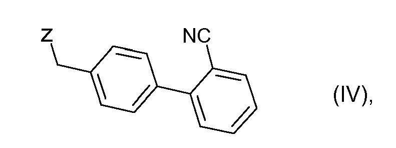 wherein Z denotes a leaving group, and the resulting compound is worked up, if desired,
(b) the cyano group of the compound 2-cyano-4'-[2"-n-propyl-4"-methyl-6"-(1"'-methyl-benzimidazol-2"'-yl)benzimidazol-1 "-ylmethyl]biphenyl thus obtained 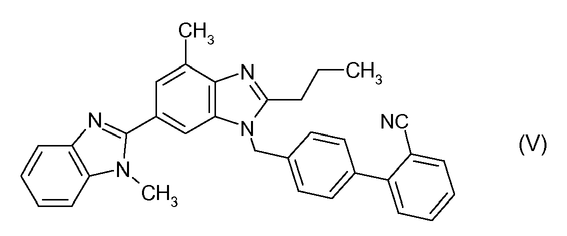 is subsequently converted into the acid function by hydrolysis in water, in an organic solvent or in a mixture of an organic solvent with water in the presence of a base at temperatures between 80 and 200°C
and the telmisartan thus obtained is converted into the hydrochloride.

2. Process according to claim 1, **characterised in that** the hydrolysis is carried out in a high-boiling solvent system selected from among ethyleneglycol/water and propyleneglycol/water at temperatures between 140 and 200°C.

3. Process according to claim 1, **characterised in that** the hydrolysis is carried out in the presence of a base selected from among lithium hydroxide, sodium hydroxide, potassium hydroxide, caesium hydroxide and calcium hydroxide or the anhydrides thereof.

4. Process according to claim 1, **characterised in that** Z denotes a halogen atom, preferably the bromine atom, or a substituted sulphonyloxy group.

5. Process according to claim 1, **characterised in that** step (a) is carried out in a solvent or mixture of solvents selected from methylene chloride, diethyl ether, tetrahydrofuran, dioxane, dimethylsulphoxide, dimethylformamide, dimethylacetamide, dimethylformamide/tert. butanol, dimethylacetamide/tert. butanol, toluene and benzene, optionally in the presence of an acid-binding agent.

6. Process according to claim 5, **characterised in that** the acid-binding agent is selected from sodium carbonate, potassium carbonate, sodium hydroxide, potassium hydroxide, sodium methoxide, potassium methoxide, potassium tert.pentoxide, potassium tert. butoxide, potassium-n-butoxide, sodium hydride, triethylamine and pyridine.

7. Process according to claim 5, **characterised in that** step (a) is carried out at a temperature between 0 and 100°C.

8. Process according to one of claims 1 to 7, **characterised in that** in step (a) the reaction of the compound (I) with a compound of general formula (IV) is carried out in a solvent or mixture of solvents selected from dimethylsulphoxide, dimethylformamide, dimethylacetamide, dimethylformamide/tert. butanol and dimethylacetamide/tert. butanol in the presence of sodium hydroxide, potassium hydroxide or potassium tert. butoxide at a temperature between 0 and 30°C.

9. Process according to one of claims 1 to 8, **characterised in that** after step (a) has been carried out the solvent is removed, the residue is treated with a solvent in which the nitrile (V) has only limited solubility or is moderately soluble in the heat, the crystals that may be precipitated after cooling are suction filtered and optionally washed and if desired the product is dried at elevated temperature.

10. Process according to claim 9, **characterised in that** the solvent for treating the residue is an alcohol, an aromatic hydrocarbon, an ether or water.

11. Process for hydrolysing the compound 2-cyano-4'-[2"-n-propyl-4"-methyl-6"-(1 "'-methylbenzimidazol-2"'-yl)benzimidazol-1 "-ylmethyl]biphenyl of formula (V) according to claim 1 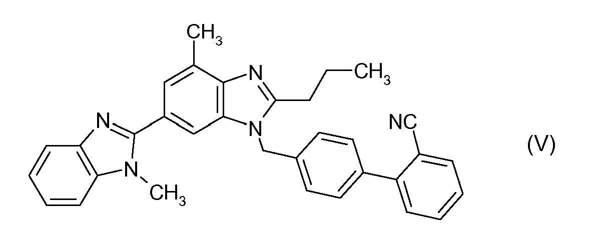 **characterised in that** the cyano group of the compound (V) is converted into the acid function in water, in an organic solvent or in a mixture of an organic solvent with water in the presence of a base at temperatures of between 80 and 200°C.

## Revendications

1. Procédé de production de chlorhydrate de telmisartan, dans lequel
(a) on fait réagir du 2-n-propyl-4-méthyl-6-(1'-méthylbenzimidazol-2'-yl)benzimidazole 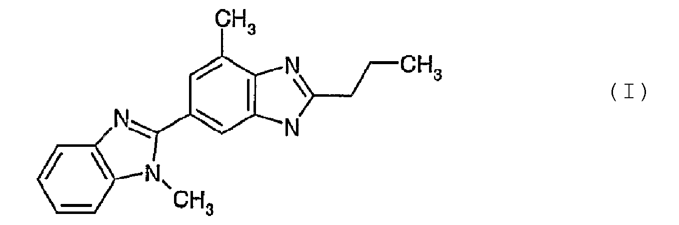 avec un composé de formule générale 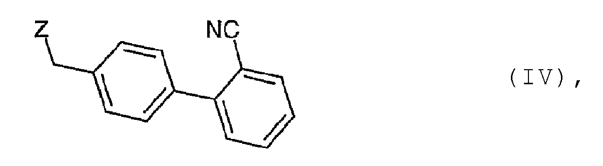 dans laquelle Z représente un groupe partant, et, si on le souhaite, on soumet le composé ainsi obtenu à un traitement,
(b) on convertit ensuite le groupe cyano du composé 2-cyano-4'-[2"-n-propyl-4"-méthyl-6"-(1"'-méthylbenzimidazol-2"'-yl)benzimidazol-1"-yl-méthyl]-biphényle ainsi obtenu 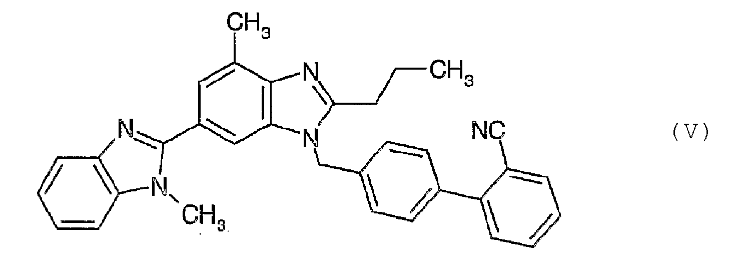 en fonction acide par hydrolyse dans de l'eau, dans un solvant organique ou dans un mélange d'un solvant organique et d'eau en présence d'une base à des températures dans la plage de 80 à 200°C
et on convertit le telmisartan ainsi obtenu en chlorhydrate.

2. Procédé selon la revendication 1, **caractérisé en ce que** l'hydrolyse se fait à des températures dans la plage de 140 à 200°C dans un système de solvants à haut point d'ébullition choisi parmi l'éthylène glycol/eau et le propylène glycol/eau.

3. Procédé selon la revendication 1, **caractérisé en ce que** l'hydrolyse se fait en présence d'une base choisie parmi l'hydroxyde de lithium, l'hydroxyde de sodium, l'hydroxyde de potassium, l'hydroxyde de césium et l'hydroxyde de calcium ou leurs anhydrides.

4. Procédé selon la revendication 1, **caractérisé en ce que** Z représente un atome d'halogène, de préférence l'atome de brome, ou un groupe sulfonyloxy substitué.

5. Procédé selon la revendication 1, **caractérisé en ce que** l'étape (a) se fait dans un solvant ou un mélange de solvants choisis parmi le chlorure de méthylène, le diéthyléther, le tétrahydro-furane, le dioxane, le diméthylsulfoxyde, le diméthylformamide, le diméthylacétamide, le diméthylformamide/tert.-butanol, le diméthylacétamide/tert.-butanol, le toluène et le benzène, le cas échéant en présence d'un agent fixant les acides.

6. Procédé selon la revendication 5, **caractérisé en ce que** l'agent fixant les acides est choisi parmi le carbonate de sodium, le carbonate de potassium, l'hydroxyde de sodium, l'hydroxyde de potassium, le méthylate de sodium, le méthylate de potassium, le tert.-pentylate de potassium, le tert.-butylate de potassium, le n-butylate de potassium, l'hydrure de sodium, la triéthylamine et la pyridine.

7. Procédé selon la revendication 5, **caractérisé en ce que** l'étape (a) se fait à une température dans la plage de 0 à 100°C.

8. Procédé selon l'une des revendications 1 à 7, **caractérisé en ce que**, à l'étape (a), la réaction du composé (I) avec un composé de formule générale (IV) se fait dans un solvant ou un mélange de solvants choisis parmi le diméthylsulfoxyde, le diméthylformamide, le diméthylacétamide, le diméthylformamide/tert.-butanol et le diméthylacétamide/tert.-butanol en présence d'hydroxyde de sodium, d'hydroxyde de potassium ou de tert.-butylate de potassium à une température dans la plage de 0 à 30°C.

9. Procédé selon l'une des revendications 1 à 8, **caractérisé en ce que**, après la réalisation de l'étape (a), on élimine le solvant, on traite le résidu dans un solvant dans lequel le nitrile (V) n'est soluble que de manière limitée ou modérément soluble à la chaleur, on aspire les cristaux éventuellement formés après le refroidissement et le cas échéant on les lave et, si on le souhaite, on sèche le produit à une température élevée.

10. Procédé selon la revendication 9, **caractérisé en ce que** le solvant utilisé pour traiter le résidu est un alcool, un hydrocarbure aromatique, un éther ou de l'eau.

11. Procédé d'hydrolyse du composé 2-cyano-4'-[2"-n-propyl-4"-méthyl-6"-(1"'-méthylbenz-imidazol-2"'-yl)benzimidazol-1"-ylméthyl]-bi-phényle de formule (V) selon la revendication 1 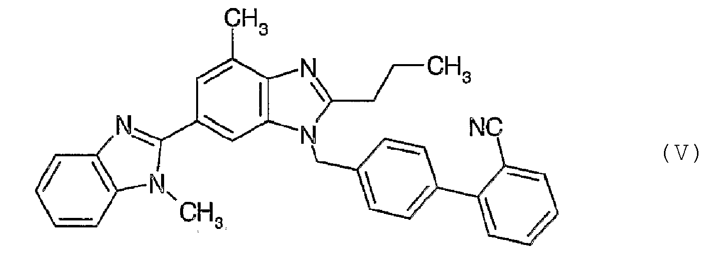 **caractérisé en ce que** le groupe cyano du composé (V) est converti en fonction acide dans de l'eau, dans un solvant organique ou dans un mélange d'un solvant organique et d'eau en présence d'une base à des températures dans la plage de 80 à 200°C.
